(19) Europäisches Patentamt
European Patent Office
Office européen des brevets

(11) **EP 2 073 016 A1**

(12) **EUROPEAN PATENT APPLICATION**

(43) Date of publication:
**24.06.2009 Bulletin 2009/26**

(51) Int Cl.:
***G01N 35/00*** *(2006.01)* ***G01N 27/72*** *(2006.01)*

(21) Application number: **07123801.8**

(22) Date of filing: **20.12.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS**<br><br>(71) Applicant: **Koninklijke Philips Electronics N.V.**<br>**5621 BA Eindhoven (NL)** | (72) Inventor: **The designation of the inventor has not yet been filed**<br><br>(74) Representative: **Van Velzen, Maaike Mathilde**<br>**Philips**<br>**Intellectual Property & Standards**<br>**P.O. Box 220**<br>**5600 AE Eindhoven (NL)** |

(54) **Magnetic label based detection**

(57)     A sensor device (100) is disclosed that is suitable for the detection of analytes in a sample (104). The sensor device (100) therefore comprises at least one sensor element (102) for sensing at the sensing surface (106) the presence and/or density of the analytes by sensing presence of magnetic or magnetizable objects. The sensor device (100) furthermore comprises a magnetic field generator (108) for generating a magnetic field having a field strength and being for controllably repelling the magnetic or magnetizable objects from the sensing surface (106). The sensor device (100) thereby is adapted for deriving the presence and/or density of analytes based on the sensed presence and/or density as function of the magnetic field.

**FIG. 1**

EP 2 073 016 A1

**Description**

FIELD OF THE INVENTION

The present invention relates to the field of sensing systems and magnetic sensor devices. More particularly the present invention relates to methods for detecting analytes in a sample, such as e.g. for detecting low-affinity binding antibodies/entities. The method and device according to the present invention may be used amongst others in biological or chemical sample analysis, in particular in diagnostics.

BACKGROUND OF THE INVENTION

Magnetic sensors based on AMR (anisotropic magneto resistance), GMR (giant magneto resistance) and TMR (tunnel magneto resistance) elements or on Hall sensors, are nowadays gaining importance. Besides the known high-speed applications such as magnetic hard disk heads and MRAM, new relatively low bandwidth applications appear in the field of molecular diagnostics, current sensing in IC's, automotive, etc. The introduction of micro-arrays or biochips comprising such magnetic sensors is revolutionizing the analysis of biomolecules such as DNA (desoxyribonucleic acid), RNA (ribonucleic acid) and proteins. Applications are, for example, human genotyping (e.g. in hospitals or by individual doctors or nurses), bacteriological screening, biological and pharmacological research. Such magnetic biochips have promising properties for, for example, biological or chemical sample analysis, in terms of sensitivity, specificity, integration, ease of use and costs.

Biochips, also called biosensor chips, biological microchips, gene-chips or DNA chips, consist in their simplest form of a substrate or surface on which a large number of different probe molecules are attached, on well defined regions on the chip, to which molecules or molecule fragments that are to be analyzed can bind if they are perfectly matched. For example, a fragment of a DNA molecule binds to one unique complementary DNA (c-DNA) molecular fragment. The occurrence of a binding reaction can be detected, for example by using markers, e.g. fluorescent markers or magnetic labels that are coupled to the molecules to be analyzed. This provides the ability to analyze small amounts of a large number of different molecules or molecular fragments in parallel, in a short time.

In a biosensor an assay takes place. Assays generally involve several fluid actuation steps, i.e. steps in which materials are brought into movement. Examples of such steps are mixing (e.g. for dilution, or for the dissolution of labels or other reagents into the sample fluid, or labeling, or affinity binding) or the refresh of fluid near to a reaction surface in order to avoid that diffusion becomes rate-limiting for the reaction. Preferably the actuation method should be effective, reliable and cheap.

In order to increase the probability and specificity of binding magnetic particles to a sensor surface, the magnetic particles may successively be attracted to and repelled from the sensor surface. According to prior art devices, this is done by applying an external magnetic field gradient in the z-direction, i.e. in a direction substantially perpendicular to the surface of the sensor device.

Switching off the gradient involves a change of field in a large volume and thus a large energy dissipation.

Furthermore, in a biosensor, it may be important to distinguish weak biomolecular bonds from strong biomolecular bonds. Even more interesting, it may be preferred to perform a population analysis, i.e. quantitatively distinguishing molecules in terms of their concentration and their binding affinity/avidity. This may, for example, be applied in the analysis of pools of antibodies in food and in medical diagnostics. In particular, there is a need in the art for sensitive devices and methods to diagnose a patient's susceptibility for immune-mediated hypersensitivity reactions. The term immune-mediated hypersensitivity is applied when an adaptive immune response occurs in an exaggerated or inappropriate form causing damage. Four types of hypersensititvity reactions have been described (Types I, II, III and IV), but in practice these types do not necessarily occur in isolation from each other. The first three types are antibody-mediated, the fourth is mediated primarily by T cells and macrophages. Especially immune-mediated drug hypersensitivity reactions (IDHR) have a significant impact on clinical practice, drug development, and public health. Hypersensitivity drug reactions are responsible for significant morbidity, mortality and socio-economic costs that are often underestimated. Drug hypersensitivity reactions represent one third of adverse drug reactions, can be life threatening and motivate changes on drug prescription. They concern more than 7% of the general population, therefore being an important public health problem.

Laboratory (or *in vitro*) tests for specific drug hypersensitivity (eg, RAST, histamine release, basophil or mast cell degranulation, lymphocyte transformation) are either unreliable or remain experimental. Diagnosis of drug hypersensitivity is difficult, as an enormous amount of different drugs can elicit various immune-mediated diseases with distinct pathomechanism. Skin tests are useful for the assessment and predictive risk of serious adverse reactions for a small number of drugs, but skin tests are limited in their utility to adverse drug reactions mediated by Type 1 hypersensitivity (IgE-mediated). One of the commonly used in-vitro tests to determine immune mediated response to drugs is the lymphocyte transformation test. The main advantage of this test is its applicability with many different drugs in different immune reactions, as drug-specific T cells are almost always involved in drug hypersensitivity reactions. Its main disadvantages are that an in-vitro proliferation of T cells to a drug is difficult to transfer to the clinical situation and that the test per se is rather cumbersome and technically demanding. So, there is a clear need for rapid diagnostic tests to determine the immune response of patients when protein based drugs are administered. Or in other words, there is a clear need for rapid and highly sensitive assays to determine the presence of specific yet weakly binding moieties such as immuno-based hypersensititvity reaction mediating antibodies.

Traditionally, in bioassays a distinction between specific and unspecific bonds is made by a washing step, but in this way it is difficult to do a population analysis, to determine the presence of weak binders and it requires careful fluid handling steps. For an integrated biosensor, the use of magnetic forces to make this distinction is more beneficial and methods for separation or isolation of biomolecules and washing on the basis of magnetic forces are known in the art. These methods largely rely on an on/off switch of the magnetic field in an attracting or repelling direction. International patent application WO 2005/111596 describes a method and device for distinguishing a specific binding from a less specific binding between a magnetic particle and a surface of another entity by applying a magnetic field and detecting a physical parameter of the magnetic particle attached to the surface.

## SUMMARY OF THE INVENTION

It is an object of the present invention to provide good magnetic sensor devices and methods for detecting analytes in a sample using magnetic or magnetizable objects. It is an advantage of embodiments according to the present invention that highly sensitive detecting of analytes can be obtained, e.g. for weak bound particles.

The above objective is accomplished by a method and device according to the present invention.

The present invention relates to a sensor device suitable for the detection of analytes in a sample, the sensor device comprising at least one sensor element for sensing at the sensing surface the presence and/or density of the analytes by sensing presence of magnetic or magnetizable objects and a magnetic field generator for generating a magnetic field having a field strength and being for controllably repelling the magnetic or magnetizable objects from the sensing surface, wherein the sensor device is adapted for deriving the presence and/or density of analytes based on the sensed presence and/or density as function of the magnetic field. The density may be an aerial density.

The sensor device further may comprise a controller for controlling the magnetic field generator to modulate the magnetic field strength in a controllable way. An advantage of these embodiments is that the sensor device may be adaptable to the purpose of the analysis, to the nature and the strength and/or flexibility of the low-affinity bonds or interactions that need to be detected.

The controller may control the magnetic field generating means to modulate the magnetic field strength by increasing the magnetic field strength as function of time. Modulation as function of time may occur in a continuous manner or in a stepwise manner, at defined intervals. Likewise, recording of a signal measured by the at least one sensor element may be continuous or stepwise. In an embodiment of the invention, the device further may comprise driving means for controlling modulation of the first magnetic field strength. According to embodiments of the invention, such driving means for controlling modulation of the first magnetic field strength may be driving means for controlling the first magnetic field generating means to control modulation of the first magnetic field strength as function of time.

The controller may provide synchronization between the magnetic field generating means and the at least one sensor element.

The sensor device further may comprise a processor for determining the presence and/or density of analytes based on a correlating of the magnetic field strength on signals recorded by the at least one sensor element. The signal may be recorded at defined intervals as function of time.

The sensor device further may comprise output means for displaying, outputting or transferring a signal representative for the number of sensed magnetic or magnetizable objects as function of the magnetic field strength. The output means may be adapted for outputting the result to a user, a readily accessible memory, another computer or a network or a computer-readable medium. The sensor device may comprise a temperature controller for controlling the temperature at which the sensor device is operated.

The sensor device further may comprise drive circuitry for driving the at least one sensor element. It is an advantage of embodiments that the sensor device can be driven as to sense at different magnetic field strengths, allowing to derive binding strengths and/or different flexibilities of certain bonds.

The at least one sensor element may be any of a magnetic sensor element, an optical sensor element, an acoustic sensor element or an electrical sensor element. The at least one sensor element may be a magnetic sensor element selected from the group consisting of GMR sensor element, TMR sensor element, AMR sensor element and Hall sensor element. The magnetic sensor device may be formed in a substrate and, according to embodiments of the invention, the at least one sensor element may be integrated in the sensor substrate. However, according to other embodiments of the invention, it is also possible that the at least one sensor element may not be integrated in the sensor substrate and that it may be partially or fully embedded in a sensor reader. As one example, the at least one sensor element may be a magnetoresistive sensor element that is embedded in the substrate. As another example, the at least one sensor element may be an optical imaging system that is embedded in the instrument for sensor readout. The at least one magnetic field generating means may be a current wire optionally integrated in the sensor device. The magnetic field generating means may be a permanent magnet. The generated magnetic field may range between 200 A/m and 2000000 A/m. Any magnetic field generating means present in the sensor device may comprise a plurality of current wires. An advantage thereof is that no high currents are required and thus less heat dissipation occurs.

The sensor device may be for use in detection of low-affinity binding entities against a target substrate. The low-affinity binding entity may be an antibody. The sensor surface may be provided with a coating for functionalization of the sensor surface with a target substrate. The target substrate may be covalently attached to the sensor substrate. Depending on the strength to be measured, the target substrate may be attached with a binding strength of at least 0.1 pN, more preferred at least 10pN, most preferred at least 1 nN. An advantage of a device as described above is that the present invention allows for the detection or diagnosis of predisposition for immune-mediated hypersensitivity reactions of a human or an animal upon administration of a known substance, thereby allowing to avoid severe complications which may well endanger a human's or animal's life and which avoids incurring unnecessary social and/or economic costs. According to an embodiment of the invention, the known substance for which predisposition for hypersensitive reactions is determined, is attached to the surface of the sensor device. As a particular embodiment, the known substance is a drug such as but not limited to a protein drug. Alternatively, the known substance is covalently linked to the magnetic or magnetizable objects. According to another embodiment of the invention, a primary antibody with known affinity/ avidity, or a secondary antibody is attached to the surface of the sensor device. Alternatively, the primary or secondary antibody is covalently linked to the magnetic or magnetizable objects. According to a particular embodiment of the invention, the known substance is covalently attached to the surface of the sensor device, and a known antibody for competition with or displacement by (a) low-affinity antibody or antibodies to be determined in a sample against the known substance, is covalently attached to the magnetic or magnetizable object. The target substrate may be a protein drug.

In a particular embodiment, the known substance may be a drug for administration to a human or an animal. Also in a particular embodiment, the low-affinity binding antibodies may be involved in the aetiology of a hypersensitivity reaction.

According to embodiments of the invention, the sensor device further may comprise a second magnetic field generating means generating a second magnetic field with a second magnetic field strength. The second magnetic field strength may be oriented in a second direction and having a second field strength being for attracting magnetic or magnetizable objects towards the surface of the magnetic sensor device. The second direction may be substantially perpendicular to the direction of the first magnetic field. The second magnetic field may be for attracting magnetic or magnetizable objects to the sensing surface or to the point of possible binding thereto. An advantage hereof is that an assay for the determination of the presence of such objects at the surface and possible binding to the surface could take less time.

The present invention also relates to a method for detecting analytes in a sample, the method comprising sensing the presence and/or density of magnetic or magnetizable objects, applying a magnetic field of a magnetic field strength and controllably repelling the magnetic or magnetizable objects, and deriving the presence and/or density of analytes based on the sensed presence and/or density of magnetic or magnetizable objects as function of the controllably repelling. The density thereby may be an aerial density. Determining the presence may comprise processing the sensed presence and/or density of magnetic or magnetizable objects as function of the controllably repelling, e.g. according to predetermined criteria.

The method further may comprise outputting a signal indicative of the sensed presence and/or density of magnetic or magnetizable objects as function of the magnetic field strength. The method may be adapted for deriving binding strengths and/or different flexibilities of certain bonds.

The controllably repelling may be modulated as function of time. The controllably repelling may comprise modulating by a controller.

The method may comprise varying the magnetic field strength a plurality of times and sensing the presence and/or density of magnetic or magnetizable objects for the plurality of varied magnetic field strengths.

The magnetic field strength may be increased as function of time.

The method may comprise synchronizing the sensing and the applying a magnetic field. The method may comprise the step of processing the signal(s) indicating detection or observations recorded by the at least one sensor element.

The analytes may be low affinity binding analytes, wherein the low affinity binding is derived from a mediator in an immune-based hypersensitivity reaction against a known substance.

The immune-based hypersensitivity reaction may be an immune-mediated drug hypersensitivity reaction (IDHR).

The drug may be a protein drug.

The presence in a sample of the mediator in an immune-based hypersensitivity reaction may be determined and the mediator may be an antibody.

The known substance may be covalently attached to the sensing surface, wherein the mediator in an immune-based hypersensitivity reaction may be an antibody, and wherein a secondary antibody may be covalently attached to the magnetic or magnetizable object. The method may comprise exposing the sensor surface with a sample comprising the mediator and exposing the sensor surface with the secondary antibody.

The known substance may be covalently attached to the sensing surface and a known antibody with known affinity for the known substance may be covalently attached to the magnetic or magnetizable object. The method may comprise exposing the sensing surface to the known antibody, after the exposing to the known antibody, exposing the sensing surface to the mediator, and, measuring the release of known antibody bound to the sensing surface upon exposure to the mediator.

The known substance may be covalently attached to the sensing surface, and a known antibody with known affinity for the known substance may be covalently attached to the magnetic or magnetizable object. The method may comprise exposing the sensing surface to the known antibody, simultaneously exposing the sensing surface to the mediator, and measuring the binding rate of the magnetic beads to the sensing surface.

The known substance may be covalently attached to the magnetic or magnetizable object, wherein the mediator in an immune-based hypersensitivity reaction may be an antibody, and wherein a secondary antibody may be covalently attached to the sensing surface. The method may comprise mixing a sample comprising the mediator with the known substance, and exposing the sensing surface with the mixture of the sample and the known substance.

The method further may comprise applying a further magnetic field for attracting magnetic or magnetizable objects to the sensing surface.

The present invention also relates to a controller for controlling a sensor device suitable for the detection of analytes in a sample, the controller being adapted for controlling at least one sensor element for sensing at the sensing surface the presence and/or density of the analytes by sensing presence of magnetic or magnetizable objects and a magnetic field generator for generating a magnetic field having a field strength and being for controllably repelling the magnetic or magnetizable objects from the sensing surface, such that the presence and/or density of analytes can be derived based on the sensed presence and/or density as function of the magnetic field.

The present invention also relates to a computer program product for executing a method for detecting analytes in a sample as described above.

The present invention furthermore relates to a machine readable data storage device storing such a computer program product or the transmission thereof over a local or wide area telecommunications network.

In one aspect, the present invention also provides a biochip comprising at least one sensor device according to embodiments of the present invention. The present invention also provides the use of the sensor device and/or biochip according to embodiments of the invention in biological or chemical sample analysis, in particular in sample analysis for the determination of the presence of low-affinity binding biological moieties or particles, e.g. antibodies such as but not limited to biological binding moieties or particles, e.g. antibodies involved in hypersensitivity reactions. The present invention also provides the use of the method according to embodiments of the present invention for determining the binding strength and/or flexibility of the binding of magnetic or magnetizable objects to a sensing surface.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

BRIEF DESCRIPTION OF THE DRAWINGS

Fig. 1 shows a schematic representation of a sensor device for detecting analytes in a sample using controllably repelling of particles according to a first embodiment of the invention.

Fig. 2 illustrates a basic concept of controllably repelling as can be used in embodiments according to the present invention.

Figs. 3 to 6 are illustrative representations of particular embodiments of the method of the invention.

Fig. 7 is an illustrative representation of a bridging assay as can be used in an embodiment of the present invention.

In the different figures, the same reference signs refer to the same or analogous elements.

DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the term bottom and the like in the description are used for descriptive purposes and not necessarily for describing relative positions. It is to be understood that the terms so used are interchangeable under appropriate circumstances and that the embodiments of the invention described herein are capable of operation in other orientations than described or illustrated herein.

The terms "generating means" and "generator" may be used interchangeably. Also the terms "controlling means" and "controller" may be used interchangeably, and "sensor" and "sensor element".

"As function of time" as used herein, refers to both in a continuous and discontinuous manner. In a discontinuous manner may be at predefined intervals, regularly or irregularly spaced.

As used herein, "protein drug" comprises any therapeutically active substance with peptidic structure ranging in size from oligopeptides to proteins. Examples include but are not limited to replacement proteins, monoclonal or polyclonal antibodies, fragments of antibodies such as Fab or Fab$^2$ fragments, SCFV, nanobodies, etc. or fusion proteins. The protein drug may be derived from naturally occurring or synthetic proteins.

As used herein, "Secondary antibody" is an antibody (or fragment thereof such as Fab or Fab$^2$ fragments, SCFV, nanobodies, etc.) directed to (part of) the constant region of antibodies of a certain species, e.g. anti-human secondary antibodies selectively recognize and bind human antibodies.

Furthermore, the present invention will be described by means of the magnetic or magnetizable objects being magnetic particles. The term magnetic particles is to be interpreted broadly such as to include any type of magnetic particles, e.g. ferromagnetic, paramagnetic, superparamagnetic, etc. as well as particles in any form, e.g. magnetic spheres, magnetic rods, a string of magnetic particles, or a composite particle, e.g. a particle containing magnetic as well as optically-active material, or magnetic material inside a nonmagnetic matrix. Optionally, the magnetic or magnetizable objects may be ferromagnetic particles which contain small ferromagnetic grains with a fast magnetic relaxation time and which have a low risk of clustering. Again, the wording used is only for the ease of explanation and does not limit the invention in any way.

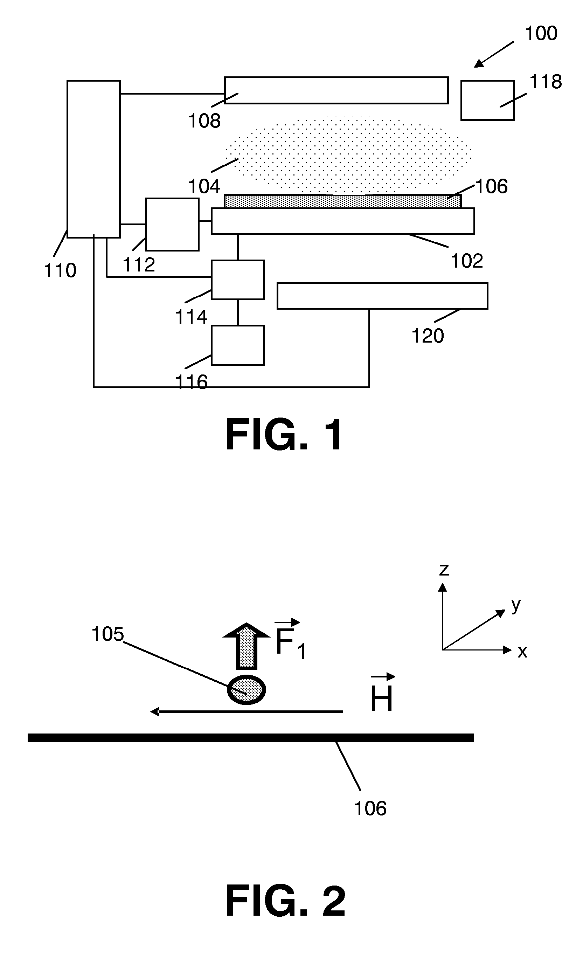

In a first aspect, the present invention provides a sensor device suitable for detecting analytes in a sample. An advantage of the sensor can be its accuracy, indeed the sensor device may be especially suitable for the detection of low-affinity binding biological moieties or particles such as antibodies or entities derived from antibodies such as antibody fragments and constructs. Such low affinity binding biological moieties or particles may include, but are not limited to, proteins (equally comprising glycosylated or phosphorylated proteins), in particular antibodies or entities derived from antibodies such as antibody fragments (Fab, etc ) and constructs, nucleic acids (equally comprising naturally occurring nucleic acids comprising DNA and RNA, as well as synthetic nucleic acid fragments such as PNA) and other biomolecules such as carbohydrates and lipids. The binding may be a protein-protein interaction, a protein-lipid, a protein-carbohydrate, a protein-nucleic acid interaction, or a nucleic acid-nucleic acid interaction. Nucleic acid-nucleic acid interactions typically comprise DNA-DNA, DNA-RNA, RNA-RNA interactions, or even nucleic acid triplex formation. In particular embodiments, lower affinity binding in nucleic acid-nucleic acid interactions may occur due to the presence of mismatches in the hybridization, or due to the limited length of the hybridizing nucleic acid fragments. In particular embodiments, the low affinity binding may occur between two proteins wherein at least one of them is an antibody directed to an epitope of e.g. a therapeutic protein or a protein drug. In a particular embodiment, the low affinity binding analytes may be proteins, peptides and in particular antibodies. A schematic representation of an exemplary sensor device 100 according to embodiments of the present invention is shown in Fig. 1. The sensor device 100 comprises at least one sensor element 102 for detecting analytes in a sample 104 by sensing at a sensing surface 106 the presence and/or density of magnetic or magnetizable objects. Such detection may be direct detection through its magnetic properties or may be indirect detection by detecting another physical parameter of the magnetic or magnetizable object or a particle coupled thereto. The sensor device 100 furthermore comprises a magnetic field generating means 108 for generating a magnetic field and having a magnetic field strength being for controllably repelling the magnetic or magnetizable objects from the sensing surface. The magnetic or magnetizable objects may be coupled to the analytes of interest, or may be coupled to reference particles used in a competition assay for detecting the analytes of interest. The sensor device 100 furthermore is adapted for deriving the presence and/or density of analytes in a sample 104 based on the sensed presence and/or density of the magnetic or magnetizable objects as function of the magnetic field strength. It is an advantage of embodiments according to the present invention that the detection technique is based on controllably repelling magnetic or magnetizable object present in a sample 104 at or near the sensing surface 106, as the latter can be used for detecting differently bound particles with high sensitivity. The system may e.g. preferably be used for detecting low affinity binding analytes. The sensor device and more particularly specific and optional components will be further described with reference to Fig. 1 by way of illustration.

As described above, the presence of magnetic or magnetizable objects at or near the sensing surface 106 in the sensor device is detected by at least one sensor element 102. The at least one sensor element 102 may sense in different ways, such as but not limited to sensing based on magnetic, optical, sonic and/or electrical detection or sensing methods. The present invention will further be described by means of a magnetic sensor device based on GMR elements. However, this is not limiting the invention in any way. The present invention may be applied to sensor devices comprising any sensor element suitable for detecting the presence or determining the amount of magnetic or magnetic or magnetizable objects, e.g. magnetic nanoparticles, on or near a sensing surface based on any property of the particles. The at least one sensor element 102 therefore can be any suitable sensor to detect the presence of magnetic particles on or near to a sensing surface, based on any property of the particles, e.g. it can detect via magnetic methods e.g. using a magnetoresistive sensor, a Hall sensor and optionally a magnetic excitation source, via optical methods e.g. using imaging, fluorescence, chemiluminescence, absorption, scattering, surface plasmon resonance, Raman, etc., via sonic, acoustic or mechanical detection e.g. using surface acoustic waves detection, bulk acoustic wave detection, cantilever based detection, quartz crystal based detection etc and/or electrical detection e.g. based on conduction, impedance, amperometric, redox cycling, etc. The magnetic or magnetizable particles therefore may have particular physical properties, such as for example luminescent properties, electrical properties, magnetic properties or acoustical properties, etc. or may be coupled to a particle having such properties. A suitable excitation means (not shown) for exciting or provoking a physical parameter to be sensed by the at least one sensor element 102 also may be present, such as e.g. an irradiation source for generating a luminescence response, a magnetic field generating means for generating a magnetic response, an acoustic wave generating means for generating an acoustic response, etc. Said at least one sensor element 102 may generate a signal reflecting such sensing at the sensing surface 106 and may be controlled do so as function of time, in a continuous or discontinuous way, in particular the at least one sensor element 102 may be controlled to generate such a signal at defined intervals as function of time. The sensor device 100 may further comprise driving means or drive circuitry 112 for driving the sensing element 102. The at least one sensor element 102 may be integrated in a sensor substrate, or may be partially or fully embedded in a sensor reader. As one example, the sensor element may be a magnetoresistive sensor element that is embedded in the substrate. As another example, the at least one sensor element 102 may be an optical imaging system that is embedded in an instrument for sensor readout.

The term "sensing surface" as used herein refers to a surface of the sensor device 100 where the determination of the magnetic or magnetizable objects takes place. Said sensing surface 106 may be a flat surface as such, or may be part of (e.g. the bottom) a reaction chamber, such as but not limited to the reaction chambers in multi-well plates. According to embodiments of the present invention, the surface of the sensor device 100 or in other words the sensing surface 106 is provided with means for derivatization or functionalization of the sensing surface 106. The surface of the sensor device may be modified by a coating which is designed to attract certain molecules or may be modified by attaching molecules to it, which are suitable to bind the target molecules which are present in the sample 104. Such molecules are known to the skilled person and may include complementary DNA, antibodies, antisense RNA, carbohydrates, etc. Such molecules may be attached to the surface by means of spacer or linker molecules. The surface of the sensor device can also be provided with molecules in the form of organisms (e.g. viruses or cells) or fractions of organisms (e.g. tissue fractions, cell fractions, membranes). The surface of biological binding can be in direct contact with the sensor chip, but there can also be a gap between the binding surface and the sensor chip. For example, the binding surface can be a material that is separated from the chip, e.g. a porous material. Such a material can be a lateral-flow or a flow-through material, e.g. comprising microchannels in silicon, glass, plastic, etc. The binding surface can be parallel to the surface of the sensor chip. Alternatively, the binding surface can be under an angle with respect to, e.g. perpendicular to, the surface of the sensor chip.

The magnetic field generating means 108 is adapted for generating a magnetic field adapted for controllably modulating the first magnetic field, or in other words, for modulating the strength of the first magnetic field in a controllable way. In a particular embodiment, the magnetic field generating means 108 provides a controllably repelling magnetic field as function of time, i.e. a magnetic field with a magnetic field strength that is variable or variably settable as function of time. Said modulation may for example be achieved by a magnetic field having an increasing the magnetic field strength as function of time. According to embodiments of the invention, the increase of the magnetic field strength as function of time may occur in a continuous way or in discrete steps, or at predetermined intervals. The magnetic field generating means 108 may be a controllable magnet, i.e. a magnet with a settable magnetic field strength. The magnetic field generating means may e.g. be an electromagnet. It may e.g. be a coil or set of coils, although the invention is not limited thereto. The magnetic field generating means 108 therefore may be adapted for repelling magnetic or magnetizable particles from the sensing surface 106. The magnetic force exerted by the generated magnetic field on a magnetic or magnetizable object, such as e.g. a super paramagnetic bead, can be given by:

$$\vec{F}_{magn} = -\nabla u = \nabla(\vec{m} \cdot \vec{B}) = (\vec{m} \cdot \nabla)\vec{B} \qquad (1)$$

with $\overline{m}$ the magnetic moment of the magnetic particle and $\overline{B}$ the applied magnetic field. According to an embodiment of the present invention, the magnetic field generating means 108 may be adapted for generating a magnetic field oriented in the xy-plane, i.e. in a horizontal direction , or in other words in a direction substantially parallel to the plane of the sensing surface 106 of the sensor device 100, hereby generating a substantially vertical repelling force or in other words a repelling force in the z-direction, i.e. when the sensing surface is lying in an xy-plane, a repelling force in a direction substantially perpendicular to the plane of the sensing surface and away from the sensing surface.

The sensor device 100 may comprise a controller 110 for controlling the magnetic field generating means 108 and the sensor device 102. The controller 110 may coordinate the functioning of the magnetic field generator 108 and the sensor 102 by synchronizing the actions of the different elements. The most appropriate way for controlling the magnetic field generating means 108 and the sensor device 102 will depend on the weak binding entities to be determined, or in other words will depend on the nature of the assay in terms of binding strength and/or binding flexibility, and/or type of assay e.g. competition assay, bridging assay, displacement assay, binding kinetics analysis etc.

As described above, the sensor device 100 is adapted for determining the presence and/or density of analytes in a sample 104. The latter can be derived based on the sensed presence and/or density of the magnetic or magnetizable objects as function of the magnetic field strength. In other words, the correlation between the sensed magnetic or magnetizable objects and the magnetic field strength can be used for determining the presence and/or density of analytes in the sample 104. The latter may be performed using a processor 114. Signals generated by the sensor element 102 may be transferred to the processor 114 which may process the signals, taking into account the controlled magnetic field strength. Such a processor 114 or processing means may facilitate or increase the sensitivity of the detection. Such processing of the signal may occur as a function of the strength of the first magnetic field, e.g. as a function of time. Said processing means 114 may be in communication with said controller 110 for receiving driving information of the sensing device and/or the magnetic field generating means for taking into account such information. The processor 114 may be adapted to operate according to a predetermined algorithm, a neural network, etc. The particular processing applied may depend on the nature of the assay in terms of binding strength and/or binding flexibility, and/or type of assay e.g. competition assay, bridge assay, displacement assay, binding kinetics analysis etc. The processor may be adapted for processing the data in agreement with the particular examples and embodiments of a method for sensing as described below. The processed data may be transferred to an output means 116, delivering the output of the measurements to the user or to a further sensor device. The output means 116 may be means for displaying, outputting or transferring the processed signal to a display, a user, a readily accessible memory, another computer on a network or a computer-readable medium. The displaying, outputting or transferring may occur prior to or after processing of the signal.

Optionally, the sensor device 100 furthermore may comprise a further magnetic field generator 120 allowing the creation of an additional magnetic field in a direction that is opposite the repelling magnetic field, thus creating a magnetic field force that will attract magnetic particles to the sensing surface 106. The latter may e.g. be used for supplying the magnetic and/or magnetizable particles to the sensing surface 106. The latter preferably is performed for efficiently providing the magnetic and/or magnetizable particles to the sensing surface. The further magnetic field generating means 120 may be adapted for generating, in combination with the first magnetic field, a magnetic field being for attracting magnetic or magnetizable objects to the surface of the sensor device. According to the embodiment, the first and additional magnetic fields may be oriented substantially antiparallel and in the xy-plane, i.e. in a direction substantially parallel to the plane of the sensing surface, hereby generating a vertical attracting force or in other words an attracting force in the z-direction, i.e. when the sensing surface is lying in an xy-plane, an attracting force in a direction substantially perpendicular to the plane of the sensing surface and towards the sensing surface. Such an attracting force on particles may increase the speed by which a binding assay using the sensor device of the present invention is performed. Such an attracting force may also allow to assist the binding of particles. Such additional attracting force may be useful in the performance of a competition assay. With substantially antiparallel is meant that the first and second direction may be not exactly opposite to each other but may include an angle of less than 10°, preferably less than 5° and most preferred less than 1°. According to the present invention, the generated fields may be homogenous or may be non-homogenous.

According to embodiments of the invention, the further magnetic field generating means may be an external magnetic field generating means. It also may be integrated in the sensor device. It may be a switchable or constant magnetic field generating means.

In some embodiments of the present invention the sensor device 100 comprises a reaction chamber that can be a disposable item to be used with a compact reader, containing the one or more magnetic field generating means and one or more detection means. Also, the device and methods of the present invention can be used in automated high-throughput testing. In this case, the reaction chamber may for example be a well plate or cuvette, fitting into an automated instrument.

The sensor device may comprise a temperature controller 118 for controlling the temperature in the sensor device, e.g. for controlling the temperature near the sensing surface.

In a second aspect, the invention provides a method for detecting analytes in a sample, e.g. low-affinity binding analytes. Such low affinity binding biological moieties or particles may include, but are not limited to, proteins (equally comprising glycosylated or phosphorylated proteins), in particular antibodies or entities derived from antibodies such as antibody fragments (Fab, etc ) and constructs, nucleic acids (equally comprising naturally occurring nucleic acids comprising DNA and RNA, as well as synthetic nucleic acid fragments such as PNA) and other biomolecules such as carbohydrates and lipids. The binding may be a protein-protein interaction, a protein-lipid, a protein-carbohydrate, a protein-nucleic acid interaction, or a nucleic acid-nucleic acid interaction. Nucleic acid-nucleic acid interactions typically comprise DNA-DNA, DNA-RNA, RNA-RNA interactions, or even nucleic acid triplex formation. In particular embodiments, lower affinity binding in nucleic acid-nucleic acid interactions may occur due to the presence of mismatches in the hybridization, or due to the limited length of the hybridizing nucleic acid fragments. In particular embodiments, the low affinity binding may occur between two proteins wherein at least one of them is an antibody directed to an epitope of e.g. a therapeutic protein or a protein drug. In a particular embodiment, the low affinity binding analytes may be proteins, peptides and in particular antibodies. The method comprises sensing the presence and/or density of magnetic or magnetizable objects. The latter preferably is done with respect to a sensing surface. The method further comprises applying a magnetic field of a magnetic field strength. Such a magnetic field may be generated by a magnetic field generator. By applying a magnetic field, magnetic or magnetizable objects can be controllably repelled, e.g. from the sensing surface. The method furthermore comprises deriving the presence and/or density of analytes based on the sensed presence and/or density of magnetic or magnetizable objects as function of said controllably repelling, i.e. for example as function of the magnetic field strength that is applied. The method of the present invention may be performed using a sensor device as described in the first aspect, although the invention is not limited thereto. Fig. 2 illustrates the situation at the sensing surface 106 when the magnetic field generator is functioning: a magnetic field B is created substantially parallel to the sensing surface 106 in the xy-plane. Consequently, a magnetic field force F is generated repelling magnetic particles away from the sensing surface, substantially in the z-direction. Particular steps of an exemplary method will further be described in more detail.

Sensing, for the purpose of the present invention also referred to as detection, may be the determination or derivation of the presence of entities with low-binding affinities, or alternatively the determination of the binding strength and/or binding flexibility and/or kinetics of binding force as such or in competition with other binding entities. Said sensing to detect the presence of magnetic particles on or near to a sensing surface, may be based on any property of the magnetic or magnetizable objects or particles coupled thereto, e.g. it may be detecting via magnetic methods e.g. using a magnetoresistive sensor, using a Hall sensor, via optical methods e.g. using imaging, using fluorescence, using chemiluminescence, using absorption or scattering measurements, using surface plasmon resonance or Raman measurements, etc., via sonic, mechanic or acoustic detection e.g. using surface acoustic wave detection, using bulk acoustic wave detection, using mechanical properties of a cantilever or using quartz crystal, or via electrical detection such as e.g. using conduction, impedance, amperometric, redox cycling properties. The sensing can occur with or without scanning of the sensing surface of the sensor device with respect to the biosensing surface. The measurement data can be derived as an end-point measurement, as well as by recording signals dynamically or intermittently. The labels present on or in the magnetic or magnetizable object may be detected directly by the sensing method. As well, the objects may be further processed prior to detection. An example of further processing is that materials are added or that the (bio) chemical or physical properties of the label are modified to facilitate detection.

In embodiments of the present invention, the magnetic field strength may be modulated for controllably repelling, e.g. as function of time. The magnetic field strength may e.g. be increased as function of time. The modulation whereby the magnetic field strength is increased as function of time may for the purpose of the present invention also be referred to as, ramping the magnetic force. The modulation may be performed in a predetermined range. Alternatively, according to complementary embodiments of the invention, said modulation may result in providing a decrease in the first magnetic field strength, or may result in reversion of the magnetic field force in the opposite direction. The method may comprise synchronizing the applying of the magnetic field and the corresponding controllably repelling and the sensing of magnetic or magnetizable objects. The synchronizing may be for sensing magnetic or magnetizable objects at a plurality predetermined magnetic field strengths, i.e. for a plurality of predetermined magnetic field strengths whereat the magnetic or magnetizable objects are controllably repelled. Such synchronization may be used for optimizing the functioning of the sensor element for sensing and the magnetic field generator for applying a magnetic field. Alternatively, synchronization may result in an adaptation or modulation of the magnetic field strength according to the signal determined by the at least one sensor element. For example, if a too high or too low signal is sensed, a further magnetic field modulation may be induced.

In an embodiment of the present invention, determining the presence and/or density of the analytes may comprise processing the signal(s) determined or recorded by the at least one sensor element. Said processing preferably is processing as function of the magnetic field strength, e.g. as function of time for a varying magnetic field, or a combination thereof.

The methods and devices of the present invention are of particular interest in biomedical applications comprising in particular diagnostics and drugs research and development. In other words, the sensor device and methods of the present invention may be used in biological and/or chemical sample analysis. For example in the development of therapeutics, the methods and devices of the present invention may be used to determine the efficacy of synthesized drugs, or for in vitro selection of a suitable drugs based on their binding affinity. Another example is the determination of reaction rates $k_{on}$ and $k_{off}$ of biomolecules with their natural ligand, e.g. antibodies with their respective antigens, both in diagnostics as well as in the development of therapeutic antibodies such as those used in oncology.

The device and method can be used with several biochemical assay types, e.g. binding/unbinding assay, sandwich assay, competition assay, displacement assay, enzymatic assay, sequential assays, bridging assays etc. as is further illustrated by the specific embodiments explained in more detail further on. The assays of the invention are by no means limited to molecular assays, also larger moieties can be detected, e.g. cells, viruses, or fractions of cells or viruses, tissue extracts, etc.

In the area of diagnostics, there is a particular utility in the determination of adverse drug effects, in particular peptide drugs as explained in detail further on.

The device and methods of this invention may be adapted for sensor multiplexing (i.e. the parallel use of different sensors and sensing surfaces), label multiplexing (i.e. the parallel use of different types of labels) and chamber multiplexing (i.e. the parallel use of different reaction chambers). The device and methods described in the present invention can be used as rapid, robust, and easy to use point-of-care biosensors for small sample volumes. In particular, by the combination of small sample volumes and suitability for multiplexing applications, the invention also provides a biochip comprising at least one sensor device according to embodiments of the present invention, or a biochip operating according to at least one method according to embodiments of the present invention.

According to a preferred embodiment of the present invention, low affinity binding can be derived e.g. from a mediator in an immune-based hypersensitivity reaction against a known substance. The low-affinity binding entity may be determined indirectly: the low-affinity binding entity to be determined in a sample may not be labeled directly, or in other words, may not be covalently attached to the label identified by the at least one sensor element. The low-affinity binding moiety may be sandwiched between its low affinity binding partner, or alternatively, the presence of the low-affinity binding entity may be detected trough a competition or displacement assay in the presence with a labeled binding competitor. With mediator in an immune-based hypersensitivity reaction is meant an element present in a sample derived from a human or animal body that plays a role in the etiology of an immune-based hypersensitivity reaction such as but not limited to antibodies, or molecules at the surface of a certain class of immune mediating cell, e.g. T-lymphocytes, surface molecules that may be responsible for the recognition of (a) antigen(s) against which the hypersensitivity reaction occurs.

In particular, the immune-based hypersensititvty reaction may be an immune-mediated drug hypersensitivity reaction (IDHR). Such IDHR may be life-threatening and usually occurs with complex drugs such as but not limited to protein drugs.

For the purpose of the present invention, protein drugs encompass replacement proteins such as recombinant proteins for restoring its function in the human body, (monoclonal) antibodies and fusion proteins. In principle any kind of protein may be possess certain antigenic properties that may induce an immune-based hypersensitivity reaction. Relatively small peptides may be referred to by the number of amino acids (e.g. di-, tri-, tetrapeptides). A peptide with a relatively small number of amide bonds may also be called an oligopeptide (up to 50 amino acids), whereas a peptide with a relatively high number (more than 50 amino acids) may be called a polypeptide or protein. In addition to being a polymer of amino acid residues, certain proteins may further be characterized by the so called quaternary structure, a conglomerate of a number of polypeptides that are not necessarily chemically linked by amide bonds but are bonded by forces generally known to the skilled professional, such as electrostatic forces and Vanderwaals forces. The terms peptides, proteins or mixtures thereof as used herein is to include all above mentioned possibilities.

Typical examples of IDHR inducing protein drugs belong to the class of hormones and growth factors such as follicle stimulating hormone, prolactin, angiogenin, epidermal growth factor, calcitonin, erythropoietin, thyrotropic releasing hormone, insulin, growth hormones, insulin-like growth factors 1 and 2, skeletal growth factor, human chorionic gonadotropin, luteinizing hormone, nerve growth factor, adrenocorticotropic hormone (ACTH), luteinizing hormone releasing hormone (LHRH), parathyroid hormone (PTH), thyrotropin releasing hormone (TRH), vasopressin, cholecystokinin, and corticotropin releasing hormone; cytokines, such as interferons, interleukins, colony stimulating factors, and tumor necrosis factors: fibrinolytic enzymes, such as urokinase, kidney plasminogen activator; and clotting factors, such as Protein C, Factor VIII, Factor IX, Factor VII and Antithrombin III.

Examples of other proteins or peptides are albumin, atrial natriuretic factor, renin, superoxide dismutase, alpha 1-antitrypsin, lung surfactant proteins, bacitracin, bestatin, cydosporine, delta sleep-inducing peptide (DSIP), endorphins, glucagon, gramicidin, melanocyte inhibiting factors, neurotensin, oxytocin, somostatin, terprotide, serum thymide factor, thymosin, DDAVP, dermorphin, Met-enkephalin, peptidoglycan, satietin, thymopentin, fibrin degradation product, des-enkephalin- alpha -endorphin, gonadotropin releasing hormone, leuprolide, alpha -MSH and metkephamid.

According to an embodiment of the invention, the method of the invention determines the presence in a sample of said mediator in an immune-based hypersensitivity reaction is determined and said mediator is an antibody. Said the immune-based hypersensitivity reaction may be directed against a known substance that is covalently attached to the sensing surface.

According to another embodiment of the invention, the method may further comprise a step of applying a second magnetic field of a second magnetic field strength for attracting magnetic or magnetizable objects to the sensing surface, generated by a second magnetic field generator. A step of attracting the magnetic or magnetizble objects to the sensing surface may enhance the binding interaction between the magnetic or magnetizble objects to the sensing surface: the attracting force may speed up the binding, or the attracting force may increase the strength of the binding interaction. The latter effect may be of particular use in optimizing a competition or displacement assay as described for particular embodiments of the invention.

By way of illustration, a number of particular embodiments are provided illustrating particular features and advantages of the aspects as described above.

According to a particular embodiment, a method as described above is provided, wherein a secondary antibody, i.e. an antibody directed to (part of) the constant region of antibodies of a certain species, is covalently attached to a magnetic or magnetizable object. The method of this particular embodiment may further comprise the steps of exposing the sensing surface with a sample comprising the mediator, exposing the sensing surface with the secondary antibody, and measuring the presence and/or density of magnetic or magnetizable objects at the sensing surface. In particular, the sensing surface may first be exposed to the sample, prior to exposure to the secondary antibody. The method of this particular embodiment may also further comprise the steps of modulating said first magnetic field strength as function of time, in particular said modulation may be increasing said first magnetic field strength (also referred to as ramping the magnetic force), and, monitoring the presence and/or density of magnetic or magnetizable objects at the sensing surface as function of said time, in particular monitoring the release of magnetic or magnetizable objects from the sensing surface for a plurality of different magnetic field strength values.

The method of this particular embodiment may also comprise the step of replacing the sample and non-bound magnetic or magnetizable particles in the neighborhood of the sensing surface by a solution without interfering antibodies. Such a step may be seen as a washing step for removing antibodies not relevant to the interaction of antibodies present in the sample with the known substance attached to the sensing surface, thereby increasing the sensitivity of the method or assay.

According to another particular embodiment, the known substance is also covalently attached to said sensing surface, and a known antibody with known affinity for said known substance is covalently attached to the magnetic or magnetizable object. According to this particular embodiment, the method may further comprise the steps of exposing the sensing surface to the known antibody attached to the magnetic or magnetizable object, and exposing the sensing surface to the sample comprising the mediator. Within said particular embodiment, the sensing surface may be exposed to the known antibody prior to exposure to the sample, and the release of known antibody bound to the sensing surface may be measured upon exposure to the sample. Measuring the release of the known antibody from the sensing surface after exposure to the sample allows to determine the presence of antibodies in the sample capable to displace the known antibody from binding with the known substance. The efficiency of such a displacement will partly depend on the affinity of the antibodies in the sample for the known substance. The efficiency of such a displacement assay may be adjusted by modulating the repelling force of the first magnetic field on the known antibody attached to a magnetic or magnetizable object. For example, displacement of the known antibody may be facilitated by ramping the magnetic force, especially in the case where the affinity for binding the known substance is lower for the antibodies in the sample compared to the affinity for binding of the known antibody. Ramping of the magnetic force may optimize the signal: a dissociation-oriented force will make the assay sensitive to small $k_{on}$. Alternatively, an association-oriented modulation of the magnetic force, i.e. reducing the repelling magnetic field strength, will make the assay more sensitive to large $k_{on}$. The magnetic force can be gradually increased as a function of time while the release of beads is monitored. The larger the $k_{on}$ of the low-affinity binding entity to be identified, the faster the magnetic or magnetizable objects will be displaced from the sensing surface.

Different species of antibodies may be identified in the sample by controlling, e.g. ramping, the magnetic force and simultaneously monitoring the release of beads from the substrate: different species of antibodies will be charac-

terized by different $k_{on}$ for binding to the known compound attached to the sensing surface. Such differences in $k_{on}$ will result in different abilities of the antibodies to displace the known antibody from the known compound. Beads with different known antibodies characterized by different binding affinities (different $k_{on}$) for the known compound may be used. Alternatively within said particular embodiment, the sensing surface may be simultaneously exposed to the known antibody and to the antibody(ies) present in the sample, and the binding rate of the magnetic or magnetizable objects to the sensing surface is measured. Measuring the binding rate and final density of the known antibody attached to a magnetic or magnetizable object to the sensing surface upon exposure to both the sample and the known antibody, allows to determine the presence of antibodies in the sample capable to compete with the known antibody for binding with the known substance. The efficiency of such a competition will partly depend on the affinity of the antibodies in the sample for the known substance ($k_{on}$), and how this affinity relates to the binding affinity of the known antibody with the known substance. The efficiency of such a competition assay may be optimized by modulating the repelling force of the first magnetic field on the known antibody attached to a magnetic or magnetizable object. For example, competition by the antibodies present in the sample with the known antibody for binding to the known compound, may be enhanced by increasing the repelling force by increasing the first magnetic field strength, especially in the case where the affinity for binding the known substance is lower for the antibodies in the sample compared to the affinity for binding of the known antibody. Beads with different known antibodies characterized by different binding affinities (different $k_{on}$) for the known compound may be used. According to another embodiment, the known substance is covalently attached to said magnetic or magnetizable object, and a secondary antibody is covalently attached to said sensing surface. The method of this embodiment may further comprise the steps of exposing the sensing surface with a sample comprising the mediator, exposing the sensing surface with the known substance, and measuring the presence and/or density of magnetic or magnetizable objects at the sensing surface. As is within the knowledge of the skilled person, said exposing of the sensing surface with the sample, and subsequently with the known compound should last as long as needed for the interaction between the antibody(ies) present in the sample and directed against the known substance, to interact with the secondary antibody, and between the antibody(ies) present in the sample and the know substance respectively. Within this embodiment the sample may first be mixed and/or incubated with the known compound attached to a magnetic or magnetizable object, subsequently exposing the mixture to the sensing surface. This would allow a washing step thereby avoiding the presence on the sensing surface of interfering antibodies. Methods for isolating magnetic or magnetizable objects but retaining entities bound to its surface are known to the skilled person. Such a washing step has the advantage to increase sensitivity of the assay. The method of this particular embodiment may also further comprise the steps of modulating said first magnetic field strength in function of time, and, monitoring the presence and/or density of magnetic or magnetizable objects at the sensing surface as function of said time. In a particular embodiment, such modulation as function of time is performed after the formation of the binding between secondary antibody and sample antibodies and between the sample antibodies and the known substance.

As is within the knowledge of the skilled person, for the above embodiments, the contact time during which bindings are formed, i.e. during the exposure of the known compound with the low-affinity binding entity, during the contact of an antibody with a secondary antibody, etc., said contact time should last as long as needed for the interaction to take place.

It is an advantage of embodiments of the present invention that the method for detecting analytes used repelling magnetic or magnetizable particulate objects, e.g. magnetic particles, to and from a sensing surface. The magnetic sensor device and the method according to the present invention can advantageously be used for distinguishing between strong and weak bonds between magnetic or magnetizable objects, e.g. magnetic particles, and a sensing surface and for distinguishing between specific, albeit weak, bonds and nonspecific bonds of magnetic or magnetizable objects, e.g. magnetic particles, on a sensing surface. Furthermore, the magnetic sensor device may be used for determining binding strength and/or binding flexibility of magnetic or magnetizable objects, e.g. magnetic particles, to a sensing surface. The sensor device may be adapted to derive based thereon characterization information. The sensor device and the method of the present invention may also be used for determining the kinetics of a binding interaction. Hence, the sensor device according to the present invention may combine in one sensor device the detection of magnetic or magnetizable objects, e.g. magnetic particles, bound to the sensing surface and, for example, the determination of the strength and/or flexibility of the bond between the magnetic or magnetizable object, e.g. magnetic nanoparticle, and the sensing surface.

In an embodiment of the present invention, the target molecules are antibodies, in particular antibodies with low binding affinity for the molecule on the sensing surface, such as but not limited to antibodies involved in the first stages of the development of a hypersensitive immune-mediated drug reaction.

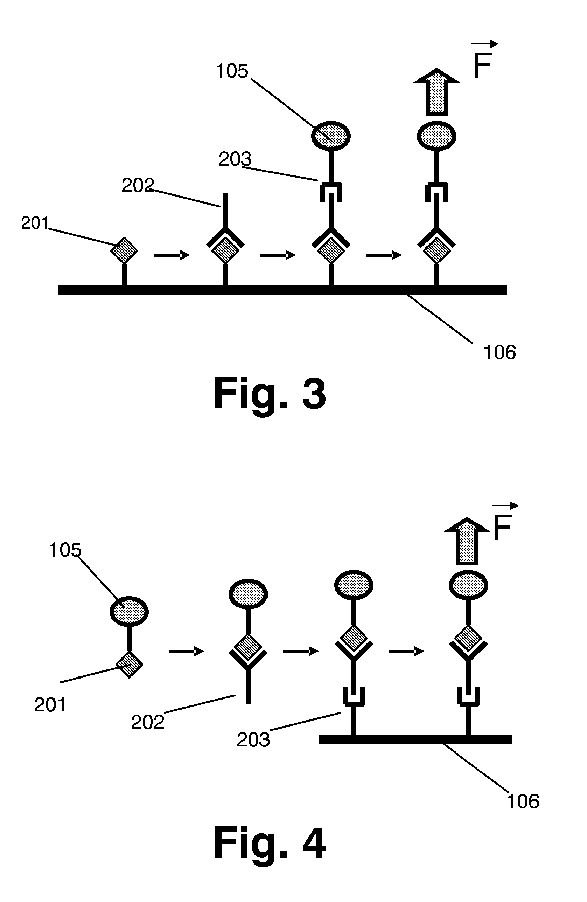

By way of example, some of the above embodiments will be further illustrated with respect to Fig. 3 to Fig. 6. Fig. 3 illustrates a binding assay whereby a known substance 201 against which low-affinity binding antibodies 202 in a sample are to be determined, is attached onto the sensing surface 106. Also used in this assay are secondary antibodies 203 which are attached to a magnetic particle 105. In a first step, the sample comprising the low-affinity binding antibodies 202 is brought into contact with the sensing surface exposing the known compounds 201, thereby allowing complex formation between the antibodies 202 and the known compound 201. A subsequent step is to expose the complexes formed to a preparation comprising a secondary antibody 203 attached to a magnetic particle 105, thereby allowing a complex to be formed between the secondary antibody 203 and the low-affinity binding antibody 202. Hence, it is only in the case where a primary antibody binds the known compound attached to the sensing surface, that the secondary antibody can attach in its turn to the sensing surface and will be retained to the sensing surface as soon as some repelling magnetic force F will be applied. The weakest link in the attachment of the magnetic particle to the sensing surface is the interaction or complex formed with the weakest binding strength, hence the low-affinity binding between the known compound and the low-affinity binding antibody. Seen the difficulty in determining low-affinity interactions, the sensitivity of the assay can be increased by ramping the first magnetic field force and thereby assessing the strength and kinetics of the binding interaction(s) responsible for the retention of the magnetic particles to the sensing surface.

Fig. 4 illustrates a particular embodiment which is very similar to the one illustrated in Fig. 3, however, the known compound is attached to the magnetic particle and the secondary antibody is attached to the sensing surface. A first step of the method comprises incubating the sample with the magnetic particles to allow the formation of a low-affinity complex between the known compound and a low-affinity antibody present in the sample. Such complexes may be easily isolated from non-relevant antibodies present in the sample by methods provided for by the prior art (CF. Background methods describing magnetic washing). A composition comprising said isolated complexes is then brought in contact with the sensing surface bound magnetic particles. The influence a repelling magnetic force F and possibilities offered by modulating the strength as function of time are similar to those for the method illustrated in Fig. 3. The additional advantage of this embodiment however, is that the sensitivity may be substantially increased by the possibilities offered by known methods for carefully washing magnetic particle whereon low-affinity binding entities are attached. Additionally, in the field of biosensor technology, only one type of surface bound secondary antibody in a biosensor device may allow for the determination of a wide range of low-affinity binding antibodies.

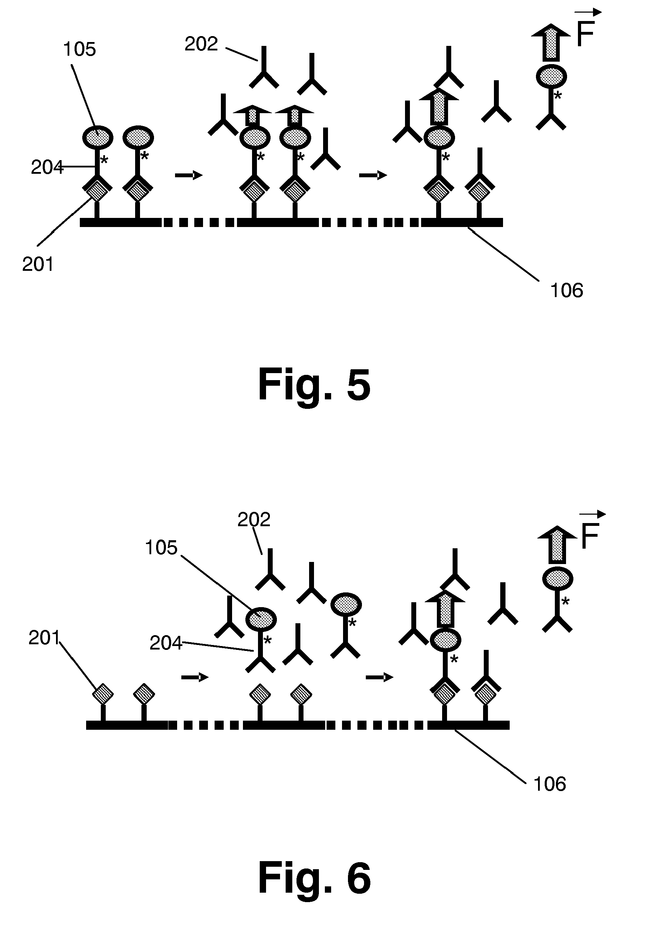

Fig. 5 illustrates a displacement assay for the determination of low-affinity binding entities in a sample, whereby a known antibody having a known low-affinity for the known compound attached to the sensing surface is first brought into contact with the sensing surface to allow a complex to be formed. Subsequently the sample is added and the release of magnetic particles from the sensing surface is measured, preferably under growing pressure of a ramping first magnetic field. Fig. 5 thereby illustrates the displacement of antibodies labeled with magnetic particles, e.g. superparamagnetic nanoparticles. Such a displacement assay can be especially suitable for antigens for which the epitope is predictable, e.g. because of a low number of surface accessible epitopes and of the presence of an immunogenic epitope.

Fig. 6 illustrates an alternative to the displacement assay, namely a competition assay between antibodies labeled with magnetic particles and antibodies from the sample. Such assays may be especially suitable for antigens for which the epitope is predictable, e.g. because of a low number of surface accessible epitopes and of the presence of an immunogenic epitope.

The advantage of the displacement and competition assay over the assays illustrated by Fig 3 and Fig. 4, is that not only low-affinity binding antibodies, but any low-affinity binding interaction with the known compound can be determined in the sample, which may for example find its particular application in drugs research, determining drug inefficiency through unanticipated interactions in the human body, etc.

By way of illustration, the present invention not being limited thereto, further exemplary embodiments are provided below, illustrating features and advantages of embodiments of the present invention.

A first particular further embodiment may be a sequential assay in which binding of an antibody from the samples takes place first, where after exposure of the binding surface to magnetic particle labeled antibodies can take place. The exposure to magnetic particle labeled antibodies may be performed either direct or after a fluid wash step.

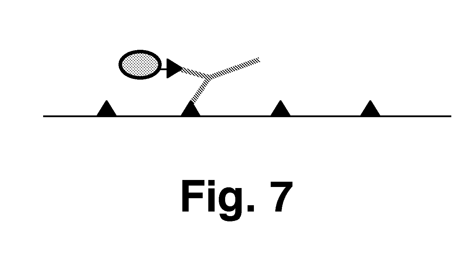

Further particular embodiments make use of a bridging assay, such as for example a bridging ELISA assay. Such assays may reduce background readings by the requirement for two specific binding events using the two antigen binding sites of the antibody. It may be e.g. especially suitable in case an antigen with multiple and unpredictable epitopes, such as e.g. in case of protein drugs, is to be detected. In applying these bridging assays in embodiments according to the present invention, the bridging assays use magnetic particles as labels. The latter is illustrated in Fig. 7. It is an advantage of embodiments using bridging ELISA assays that the species specific secondary antibodies are not needed and the epitopes against which the antibody of interest is directed does not need to be known.

In one aspect, the present invention also relates to a controller for controlling the magnetic field generator and the sensing element such that magnetic or magnetizable particles are sensed as function of a magnetic field strength of a magnetic field in order to derive presence and/or density of analytes in a sample on a sensing surface. The controller may be especially suitable for operation with a sensor device as described in the first aspect. The controller may comprise a buffer memory and may send control signals to the driving circuitry for the sensing element and/or to the magnetic field generating means. The controller may comprise software or hardware means for controlling the sensor element and/or the magnetic field generating means. The controller may be used for setting the values of the magnetic field strength of the magnetic field generated by the magnetic field generator. The controller may also be adapted to control the operation of the sensor device. The controller may use data to drive the magnetic field generator and/or sensing element. The controller may be programmable, e.g. it may include a microprocessor or an FPGA. In accordance with embodiments of the present invention a sensor device in accordance with the present invention may be programmed. Accordingly, the present invention includes a computer program product which provides the functionality of any of the methods according to the present invention when executed on a computing device. Further, the present invention includes a data carrier such as a CD-ROM or a diskette which stores the computer product in a machine readable form and which executes at least one of the methods of the invention when the program stored on the data carrier is executed on a computing device. Nowadays, such software is often offered on the Internet or a company Intranet for download, hence the present invention includes transmitting the computer product according to the present invention over a local or wide area network. The computing device may include a personal computer or a work station. The computing device may include one of a microprocessor and an FPGA.

**Claims**

1. A sensor device (100) suitable for the detection of analytes in a sample (104), the sensor device (100) comprising

   - at least one sensor element (102) for sensing at the sensing surface (106) the presence and/or density of the analytes by sensing presence of magnetic or magnetizable objects,
   - a magnetic field generator (108) for generating a magnetic field having a field strength and being for controllably repelling the magnetic or magnetizable objects from the sensing surface (106),

   wherein the sensor device (100) is adapted for deriving the presence and/or density of analytes based on the sensed presence and/or density as function of the magnetic field.

2. A sensor device (100) according to claim 1, the sensor device (100) further comprising a controller (110) for controlling said magnetic field generator (108) to modulate the magnetic field strength in a controllable way.

3. A sensor device (100) according to claim 2, wherein the controller (110) controls said magnetic field generating means (108) to modulate the magnetic field strength by increasing said magnetic field strength as function of time.

4. A sensor device (100) according to any of claims 2 to 3, wherein the controller (110) provides synchronisation between said magnetic field generating means (108) and said at least one sensor element (102).

5. A sensor device (100) according to any of claims 1 to 4, wherein the sensor device (100) further comprises a processor for determining the presence and/or density of analytes based on a correlating of the magnetic field strength on signals recorded by the at least one sensor element (102).

6. A sensor device (100) according to any of claims 1 to 5, wherein said at least one sensor element (102) is any of a magnetic sensor element, an optical sensor element, an acoustic sensor element or an electrical sensor element.

7. A sensor device (100) according to any of claims 1 to 6 for use in detection of low-affinity binding entities against a target substrate.

8. A sensor device according to claim 7 wherein said target substrate is a protein drug.

9. A method for detecting analytes in a sample, the method comprising

   - sensing the presence and/or density of magnetic or magnetizable objects, applying a magnetic field of a magnetic field strength and controllably repelling said magnetic or magnetizable objects, and
   - deriving the presence and/or density of analytes based on the sensed presence and/or density of magnetic or magnetizable objects as function of said controllably repelling.

10. A method according to claim 9, the method further comprising outputting a signal indicative of the sensed presence and/or density of magnetic or magnetizable objects as function of the magnetic field strength.

11. A method according to any of claims 9 to 10, wherein said analytes are low affinity binding analytes, wherein said low affinity binding is derived from a mediator in an immune-based hypersensitivity reaction against a known substance.

12. A method according to claim 11 wherein said immune-based hypersensitivity reaction is an immune-mediated drug hypersensitivity reaction (IDHR).

13. A method according to claim 12 wherein said drug is a protein drug.

14. A method according to any of claims 9 to 13, wherein said known substance is covalently attached to said sensing surface, wherein said mediator in an immune-based hypersensitivity reaction is an antibody, and wherein a secondary antibody is covalently attached to said magnetic or magnetizable object, the method comprising exposing the sensor surface with a sample comprising the mediator and exposing the sensor surface with the secondary antibody.

15. A method according to any of claims 9 to 14, wherein said known substance is covalently attached to said sensing surface, and wherein a known antibody with known affinity for said known substance is covalently attached to said magnetic or magnetizable object, the method comprising

- exposing the sensing surface to the known antibody,
- after said exposing to the known antibody, exposing the sensing surface to the mediator, and,
- measuring the release of known antibody bound to the sensing surface upon exposure to the mediator.

16. A controller for controlling a sensor device (100) suitable for the detection of analytes in a sample (104), the controller being adapted for controlling at least one sensor element (102) for sensing at the sensing surface (106) the presence and/or density of the analytes by sensing presence of magnetic or magnetizable objects and a magnetic field generator (108) for generating a magnetic field having a field strength and being for controllably repelling the magnetic or magnetizable objects from the sensing surface (106), such that the presence and/or density of analytes can be derived based on the sensed presence and/or density as function of the magnetic field.

17. A computer program product for executing a method according to any of claims 9 to 15.

**FIG. 1**

**FIG. 2**

**Fig. 3**

**Fig. 4**

**Fig. 5**

**Fig. 6**

# Fig. 7

European Patent Office

# EUROPEAN SEARCH REPORT

Application Number

EP 07 12 3801

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 180 418 B1 (LEE GIL U [US]) 30 January 2001 (2001-01-30) * column 4, line 61 - column 5, line 50; figure 4 * * column 8, lines 8-49 * * column 9, line 53 - column 10, line 54 * ----- | 1-17 | INV. G01N35/00 G01N27/72 |
| X | WO 2006/079998 A (KONINKL PHILIPS ELECTRONICS NV [NL]; KAHLMAN JOSEPHUS A H M [NL]; THIL) 3 August 2006 (2006-08-03) * page 22, line 13 - page 24, line 24; figures 1-28 * ----- | 1-17 | |
| X | US 2006/205093 A1 (PRINS MENNO W J [NL]) 14 September 2006 (2006-09-14) * paragraphs [0003], [0056], [0089], [0111] - [0135]; figure 5 * ----- | 1-17 | |
| X | WO 2007/129279 A (KONINKL PHILIPS ELECTRONICS NV [NL]; VAN LANKVELT PETRUS JOHNNES WI [N) 15 November 2007 (2007-11-15) * page 5, line 3 - page 7, line 7 * ----- | 1,9,16, 17 | TECHNICAL FIELDS SEARCHED (IPC) G01N G01R |
| X | US 2007/172890 A1 (PRINS MENNO W J [NL] ET AL) 26 July 2007 (2007-07-26) * paragraphs [0003], [0005], [0064], [0123], [0127] * ----- | 1,9,16 | |
| E | WO 2008/010110 A (KONINKL PHILIPS ELECTRONICS NV [NL]; PRINS MENNO WILLEM JOSE [NL]; KAH) 24 January 2008 (2008-01-24) * the whole document * ----- | 1,9,16, 17 | |

-/--

The present search report has been drawn up for all claims

| Place of search | Date of completion of the search | Examiner |
|---|---|---|
| The Hague | 8 April 2008 | Cantalapiedra, Igor |

EPO FORM 1503 03.82 (P04C01)

**European Patent Office**

**EUROPEAN SEARCH REPORT**

Application Number

EP 07 12 3801

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/010543 A (KONINKL PHILIPS ELECTRONICS NV [NL]; KAHLMAN JOSEPHUS A H M [NL]; DE B) 3 February 2005 (2005-02-03) * page 9, line 14 - page 10, line 26; figure 5 * ----- | 11-14 | |
| A | WO 2006/134546 A (KONINKL PHILIPS ELECTRONICS NV [NL]; PRINS MENNO W J [NL]; IMMINK ALBE) 21 December 2006 (2006-12-21) * the whole document * ----- | 11-14 | |
| A | US 2005/087000 A1 (COEHOORN REINDER [NL] ET AL COEHOORN REINDER [NL] ET AL) 28 April 2005 (2005-04-28) * figures 1-12 * ----- | 11-14 | |
| A | WO 2005/116661 A (KONINKL PHILIPS ELECTRONICS NV [NL]; KAHLMAN JOSEPHUS A H M [NL]; PRIN) 8 December 2005 (2005-12-08) * page 9, line 9 - page 11, line 22 * ----- | 1,9,16 | TECHNICAL FIELDS SEARCHED (IPC) |
| A | US 5 646 001 A (TERSTAPPEN LEON W M M [US] ET AL) 8 July 1997 (1997-07-08) * the whole document * ----- | 11-14 | |

The present search report has been drawn up for all claims

| Place of search | Date of completion of the search | Examiner |
|---|---|---|
| The Hague | 8 April 2008 | Cantalapiedra, Igor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 12 3801

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2008

| Patent document cited in search report | | Publication date | Patent family member(s) | | Publication date |
|---|---|---|---|---|---|
| US 6180418 | B1 | 30-01-2001 | AU | 762294 B2 | 19-06-2003 |
| | | | AU | 2560199 A | 02-08-1999 |
| | | | CA | 2318581 A1 | 22-07-1999 |
| | | | DE | 69934091 T2 | 21-06-2007 |
| | | | EP | 1049808 A1 | 08-11-2000 |
| | | | JP | 2002509242 T | 26-03-2002 |
| | | | WO | 9936577 A1 | 22-07-1999 |
| WO 2006079998 | A | 03-08-2006 | CN | 101111769 A | 23-01-2008 |
| US 2006205093 | A1 | 14-09-2006 | CN | 1829916 A | 06-09-2006 |
| | | | WO | 2005010527 A1 | 03-02-2005 |
| | | | JP | 2007500346 T | 11-01-2007 |
| | | | KR | 20060052889 A | 19-05-2006 |
| WO 2007129279 | A | 15-11-2007 | NONE | | |
| US 2007172890 | A1 | 26-07-2007 | CN | 1957251 A | 02-05-2007 |
| | | | WO | 2005111596 A1 | 24-11-2005 |
| | | | JP | 2007538252 T | 27-12-2007 |
| WO 2008010110 | A | 24-01-2008 | NONE | | |
| WO 2005010543 | A | 03-02-2005 | NONE | | |
| WO 2006134546 | A | 21-12-2006 | NONE | | |
| US 2005087000 | A1 | 28-04-2005 | AT | 326697 T | 15-06-2006 |
| | | | AU | 2002366904 A1 | 09-07-2003 |
| | | | CN | 1608206 A | 20-04-2005 |
| | | | DE | 60211555 T2 | 22-02-2007 |
| | | | WO | 03054523 A2 | 03-07-2003 |
| | | | JP | 2005513475 T | 12-05-2005 |
| | | | US | 2006128035 A1 | 15-06-2006 |
| WO 2005116661 | A | 08-12-2005 | CN | 1957257 A | 02-05-2007 |
| | | | JP | 2008500548 T | 10-01-2008 |
| | | | US | 2008024118 A1 | 31-01-2008 |
| US 5646001 | A | 08-07-1997 | NONE | | |

EPO FORM P0459

For more details about this annex : see Official Journal of the European Patent Office, No. 12/82

**REFERENCES CITED IN THE DESCRIPTION**

**Patent documents cited in the description**

- WO 2005111596 A